# EUROPEAN PATENT SPECIFICATION

(11) **EP 1 740 167 B1**
(45) Date of publication and mention of the grant of the patent: **28.08.2013**
(21) Application number: 05746462.0
(22) Date of filing: 29.03.2005
(51) Int. Cl.: A61K 48/00, C07H 21/04, A61K 39/395, A61K 31/42, C07K 16/24

(54) **TREATMENT OF TYPE 1 DIABETES WITH INHIBITORS OF MACROPHAGE MIGRATION INHIBITORY FACTOR**
BEHANDLUNG VON DIABETES TYP 1 MIT HEMMERN DES MAKROPHAGEN-MIGRATIONSHEMMENDEN FAKTORS
TRAITEMENT DU DIABETE DE TYPE 1 A L'AIDE D'INHIBITEURS DU FACTEUR D'INHIBITION DE LA MIGRATION DES MACROPHAGES

(30) Priority: 29.03.2004 US 557169 P
(43) Date of publication of application: 10.01.2007
(73) Proprietor: The Feinstein Institute for Medical Research, Manhasset, NY 11030 (US)
(72) Inventor: AL-ABED, Yousef, Locust Valley, NY 11560 (US)
(74) Representative: Cohausz & Florack
(86) International application number: PCT/US2005/010521
(87) International publication number: WO 2005/094338

(56) References cited:
- WO-A-01/32606
- WO-A-01/64749
- WO-A-02/20758
- WO-A-02/100332
- WO-A-03/104203
- US-A- 6 080 407
- US-A1- 2003 195 194
- US-A1- 2003 195 194
- CVETKOVIC I ET AL: "CRITICAL ROLE MACROPHAGE MIGRATION INHIBITORS FACTOR MIF ACTIVITY IN EXPERIMENTAL AUTOIMMUNE DIABETES" ENDOCRINOLOGY, BALTIMORE, MD, US, vol. 146, no. 7, 1 July 2005 (2005-07-01), pages 2942-2951, XP009067024 ISSN: 0013-7227
- BOJUNGA J ET AL: "Macrophage migration inhibitory factor and development of type-1 diabetes in non-obese diabetic mice" CYTOKINE 20030221 GB, vol. 21, no. 4, 21 February 2003 (2003-02-21), pages 179-186, XP007905223 ISSN: 1043-4666
- DATABASE BIOSIS [Online] BIOSCIENCES INFORMATION SERVICE, PHILADELPHIA, PA, US; March 2004 (2004-03), AL-ABED YOUSEF ET AL: "MIF antagonists are anti-diabetogenic." XP009103391 Database accession no. PREV200600067112 & ABSTRACTS OF PAPERS AMERICAN CHEMICAL SOCIETY, vol. 227, no. Part 2, March 2004 (2004-03), page U40, 227TH NATIONAL MEETING OF THE AMERICAN-CHEMICAL SOCIETY; ANAHEIM, CA, USA; MARCH 28 -APRIL 01, 2004 ISSN: 0065-7727 -& ANONYMOUS: "227th ACS meeting, Anaheim, CA" INTERNET ARTICLE, [Online] 2 February 2004 (2004-02-02), XP002488994 Internet - www. chemistry.org Retrieved from the Internet: URL:http://web.archive.org/web/20040401162 918/www.chemistry.org/portal/a/c/s/1/acsdi splay.html?DOC=meetings%5canaheim2004%5cin dex.html> [retrieved on 2008-07-17]
- H. LAN ET AL: 'Distinguishing Covariation From Causation in Diabetes: A Lesson From the Protein Disulfide Isomerase mRNA Abundance Trait' DIABETES vol. 53, no. 1, 01 January 2003, pages 240 - 244, XP055037754 DOI: 10.2337/diabetes.53.1.240 ISSN: 0012-1797

## Description

### BACKGROUND OF THE INVENTION

### (1) Field of the Invention

The present invention generally relates to diabetes treatment. More specifically, the invention is directed to the use of inhibitors of macrophage migration inhibitory factor for treatment or prevention of type 1 diabetes.

### (2) Description of the Related Art

US 6 080 487 describes the development of anti-MIF monoclonal antibodies provides a specific means for disrupting the mechanism by which MIF exerts its biological activity. The anti-MIF monoclonal antibodies may be used as a therapeutic for conditions involving MIF-mediated adverse effects. Cytokine 21(2003)179-186 asserts that MIF may play a possible role in autoimmune-inflammatory events such as type 1 diabetes. This asserted role was based on preliminary studies in which it was shown that MIF mRNA expression was elevated in the splenic lymphocytes of NOD mice in which diabetes was spontaneously induced. However, the MIF-protein levels in the diabetic animals where less than in normal controls. WO 01/64749 A2 describes the production of a variety of MIF antibodies, and speculates that the antibodies can be used to treat inflammatory diseases such as asthma, rheumatoid arthritis or nephritis. WO 01/32606 A1 describes the use of small molecule MIF inhibitors for treating a laundry list of diseases such as inflammatory diseases, autoimmune diseases, arthritis, ARDS, EAE, sepsis, septic shock, tumor grown and neovascularization. WO 02/100332 A2 describes small molecule MIF inhibitors and specifically isooxazoline-based molecules.

Type 1 diabetes mellitus (type 1 DM) is a multifactorial syndrome caused by the lack of endogenous insulin, thought to be due to an immune attack mediated by autoreactive T cells and macrophages against pancreatic β-cells. The disease afflicts approximately 4 million people in North America and epidemiological data concur that the incidence and thus the prevalence of the disease is increasing worldwide (I). Extensive research efforts have greatly expanded understanding of disease pathogenesis, and have revealed a critical role for several pro-inflammatory mediators. However, no effective anti-inflammatory therapeutics have been approved for the clinical management of type 1 DM. Several animal models of the disease have enhanced understanding of the molecular events that underlie the pathogenesis of diabetes. Multiple low doses of streptozotocin to susceptible strains of mice, for example, induces a diabetic condition with many of the hallmarks of human type 1 DM. Clinical and histoimmunological similarities include the development of hyperglycemia associated with infiltration of the pancreatic islets by T lymphocytes and macrophages (insulitis) (2,3). Proinflammatory cytokines, including interleukin (IL)-1β, interferon (IFN)-γ, tumor necrosis factor (TNF)-α and IL-18 play important roles in the development of streptozotocin-induced diabetes (4-7). However, administration of either recombinant IL-1β, IFN-γ, or TNF-α, or specific inhibitors of their activity, have complex and often contradictory effects on disease development and/or course, depending on animal model used, as well as on timing of administration (8-11).

The key pathogenic role played by the immune system in the pathogenesis of type 1 DM has recently focused much attention on identifying immunotherapeutical approaches that may allow halting or delaying β-cell destruction in prediabetic individuals or in those patients with newly diagnosed disease (12). Macrophage migration inhibitory factor (MIF) is a critical cytokine in local and systemic inflammation, but its role in diabetes has not been explored thoroughly. MIF is a pleiotropic cytokine produced during immune responses by activated T cells, macrophages and a variety of nonimmune cells (13,14). It acts as a critical mediator of host defense, and is being explored as a therapeutic target in septic shock as well as chronic inflammatory and autoimmune diseases (15-17). Elevated MIF gene expression has been detected in spontaneously non-obese diabetic (NOD) mice (18), but its importance in the pathogenesis of type 1 DM is unclear.

There is thus a need for further investigation into the precise role and interactions of cytokines, in particular MIF, in type 1 diabetes. The present invention addresses that need.

### SUMMARY OF THE INVENTION

Accordingly, the inventors have discovered that inhibition of macrophage migration inhibitory factor (MIF) attenuates type 1 diabetes.

Subject-matter of the invention is an antibody that inhibits macrophage migration inhibitory factor (MIF) for use in treating a mammal having type 1 diabetes or at risk for type 1 diabetes to prevent or attenuate the development of type 1 diabetes.

### BRIEF DESCRIPTION OF THE DRAWINGS

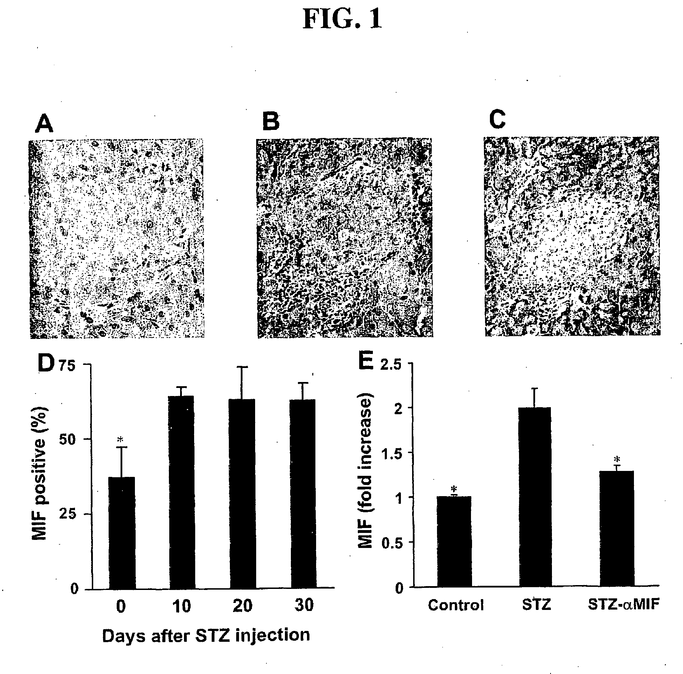

FIG. 1 is micrographs and graphs demonstrating elevated MIF protein expression in pancreas and peritoneal cells of diabetic mice. Panels A-C are light immunomicrographs of pancreata. MIF is weakly expressed by islet cells of non-diabetic control mice (Panel A); in pancreatic islets of day 10 diabetic mice, there is massive mononuclear cell (MNC) infiltration and MIF expression is markedly up-regulated (Panel B); negative staining in a serial section of diabetic mice islet (Panel C). Panel D shows a graph of quantitative image analysis showing different stages of MIF expression by islet cells from diabetic versus non-diabetic control mice. Shown are the mean ± SD of the percentage of MIF⁺ cells per islet (n = 3 mice per group). Panel E is a graph of quantitative analysis of intracellular expression of MIF protein in peritoneal cells of non-diabetic mice (Control), MLD-STZ diabetic mice (STZ), and MLD-STZ diabetic mice treated with anti-MIF antibody (STZ-αMIF), measured by cell-based ELISA performed with MIF-specific antibodies, as described in Materials and Methods of the Example. The results are presented as fold increase of control absorbance value (OD 492 nm 0.687 ±0.013). *p<0.05 refers to otherwise untreated MLD-STZ diabetic animals.

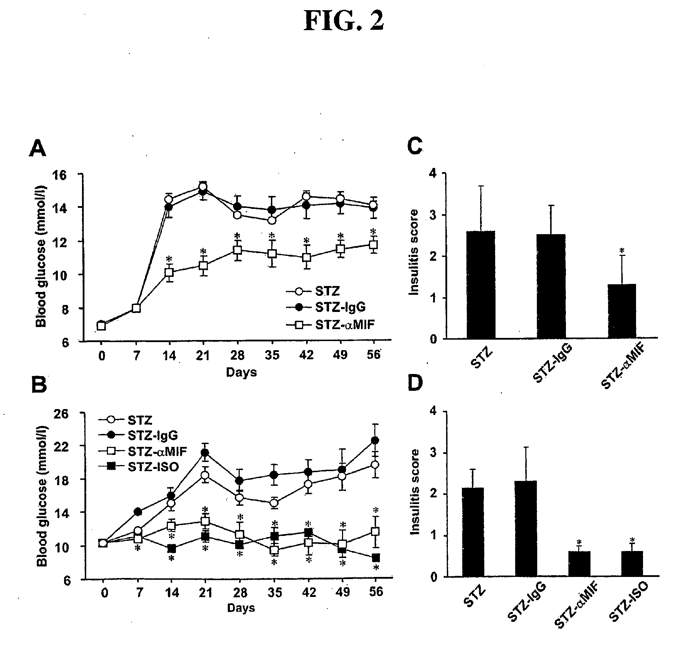

FIG. 2 is graphs of experimental results demonstrating that MIF blockade with anti-MIF antibody and ISO-1 suppress the development of hyperglycemia and insulitis. Blood glucose levels were determined in C57B1/6 mice (Panel A) and CBA/H mice (Panel B) starting at the beginning of the STZ treatment and continued through weekly measurements. Animals received MLD-STZ injections and were treated with either vehicle (STZ), non-immune rabbit IgG (STZ-IgG), anti-MIF IgG (STZ-αMIF), or ISO-1 (STZ-ISO). Histopathology analysis of pancreata from C57B1/6 mice (Panel C) and CBA/H mice (Panel D) are presented as insulitis score, as described in as described in Materials and Methods of the Example. *p<0.05 refers to corresponding STZ or STZ-IgG animals.

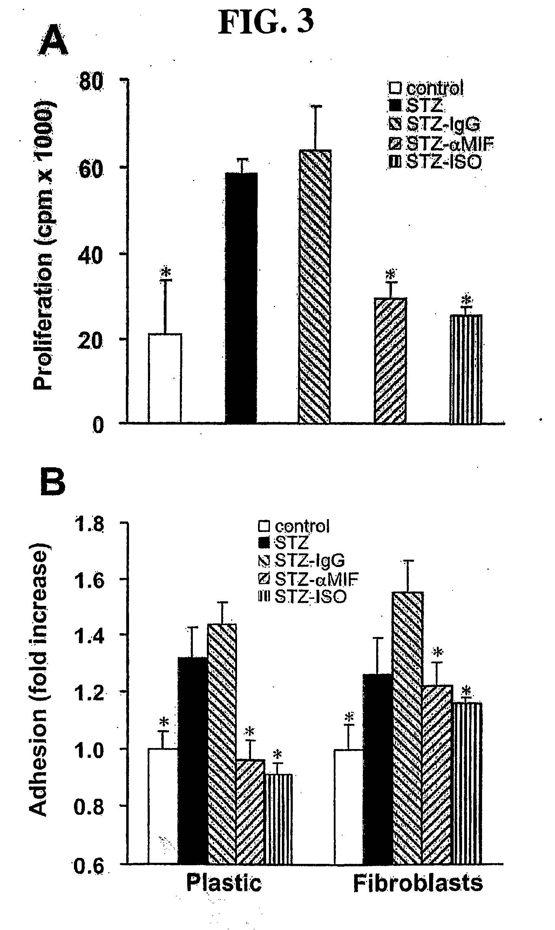

FIG. 3 is graphs of experimental results demonstrating that neutralization of MIF activity reduces splenocyte proliferation and adherence. Panel A shows ³H-thymidine incorporation, as a measure of cell proliferation, as determined in SMNC isolated from mice untreated with STZ (control), treated with STZ and vehicle (STZ), with STZ and non-immune rabbit IgG (STZ-IgG), STZ and anti-MIF IgG (STZ-αMIF), or STZ and ISO-1 (STZ-ISO). Panel B shows adhesion to plastic surface, or L929 fibroblast, as determined for SMNC isolated from the same groups of mice. The results are presented as fold increase of control adhesion to plastic surface, or to L929 fibroblasts (O.D, 570 nm 0.316 ± 0.018 and 0.905 ± 0.077, respectively). *p<0.05 refers to corresponding STZ or STZ-IgG animals.

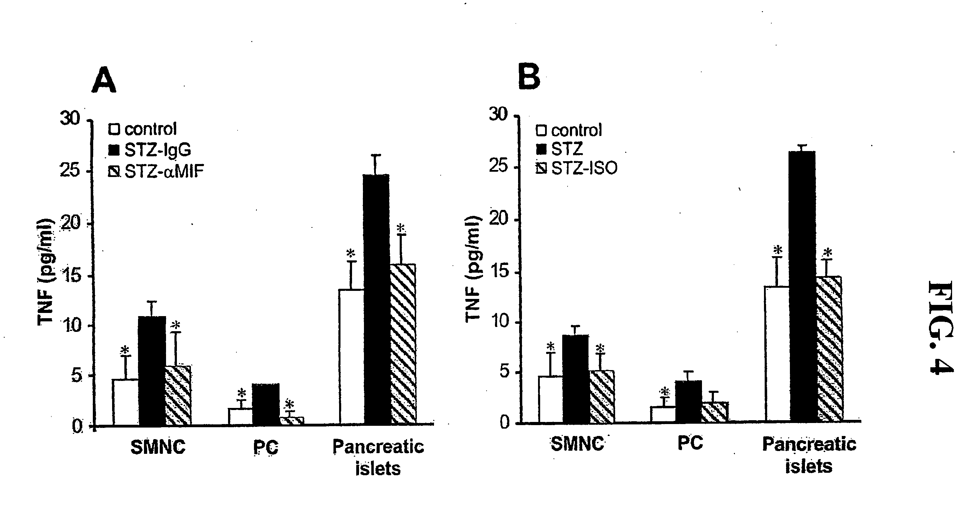

FIG. 4 is graphs of experimental results demonstrating that neutralization of MIF activity reduces the production of TNF-α. Spleen MNC (SMNC), peritoneal cells (PC) and pancreatic islets were isolated from control untreated mice (control), mice treated with STZ and non-immune rabbit IgG (STZ-IgG), STZ and anti-MIF IgG (STZ-αMIF), STZ and vehicle (STZ), and STZ and ISO-1 (STZ-ISO). TNF production was measured in the cell culture supernatants as described in Materials and Methods of the Example. Results are representative of three independent experiments with similar results. *p< 0.05 refers to corresponding STZ-IgG (Panel A) or STZ (Panel B) animals.

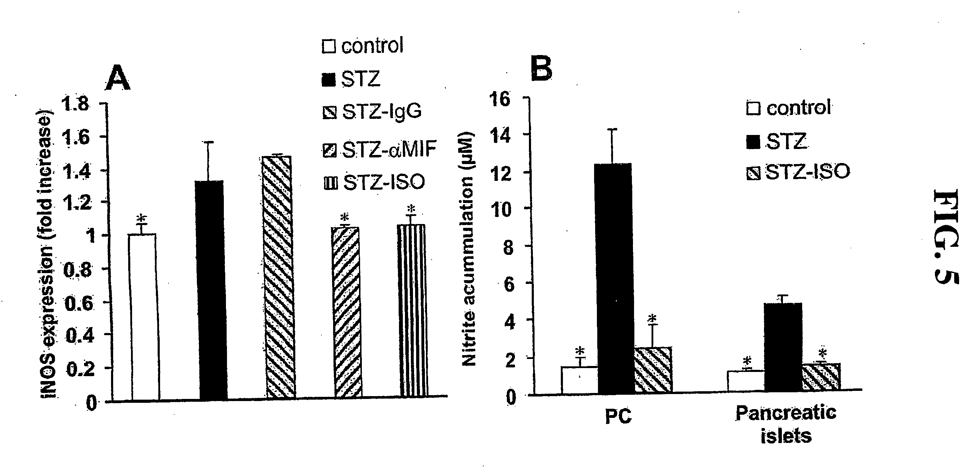

FIG. 5 is graphs of experimental results demonstrating that neutralization of MIF activity down-regulates the expression of iNOS and NO production. Peritoneal cells (PC) and pancreatic islets were isolated from mice treated as described in FIG. 3. In Panel A, iNOS expression was determined by cell-based ELISA, and presented as fold increase compared to control value (O.D. 492 nm 0.445 ± 0.027). In Panel B, after isolation from mice, PC were cultivated in medium for 48 hours, and pancreatic islets in the presence of 250 U/ml IFN-γ + IL-1β for 72 hours. Subsequently, nitrite accumulation in cell culture supernatants was determined. Results are representative of three independent experiments with similar results. *p< 0.05 refers to corresponding STZ or STZ-IgG animals.

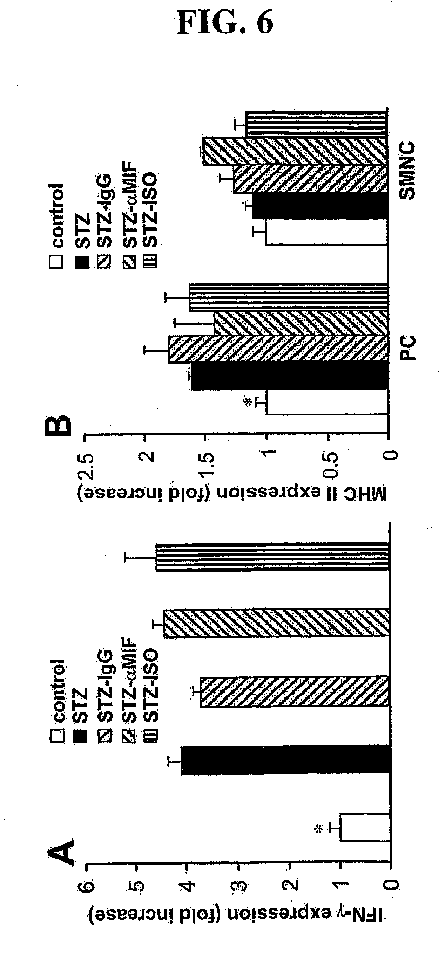

FIG. 6 is graphs of experimental results demonstrating that neutralization of MIF activity does not affect the expression of IFN-γ and MHC class II. Spleen MNC (SMNC) and peritoneal cells (PC) were isolated from mice treated as described in FIG. 3. In Panel A, IFN-γ expression in SMNC was determined by cell-based ELISA, and presented as fold increase compared to control value (O.D. 492 nm 0.070 ± 0.014). In Panel B, MHC II molecules expression in PC and SMNC was determined by cell-based ELISA, and presented as fold increase compared to control value (O.D. 492 nm 1.678 ± 0.151 and 1.204 ± 0.124, respectively). Results are representative of three independent experiments with similar results. *p< 0.05 refers to corresponding STZ or STZ-IgG animals.

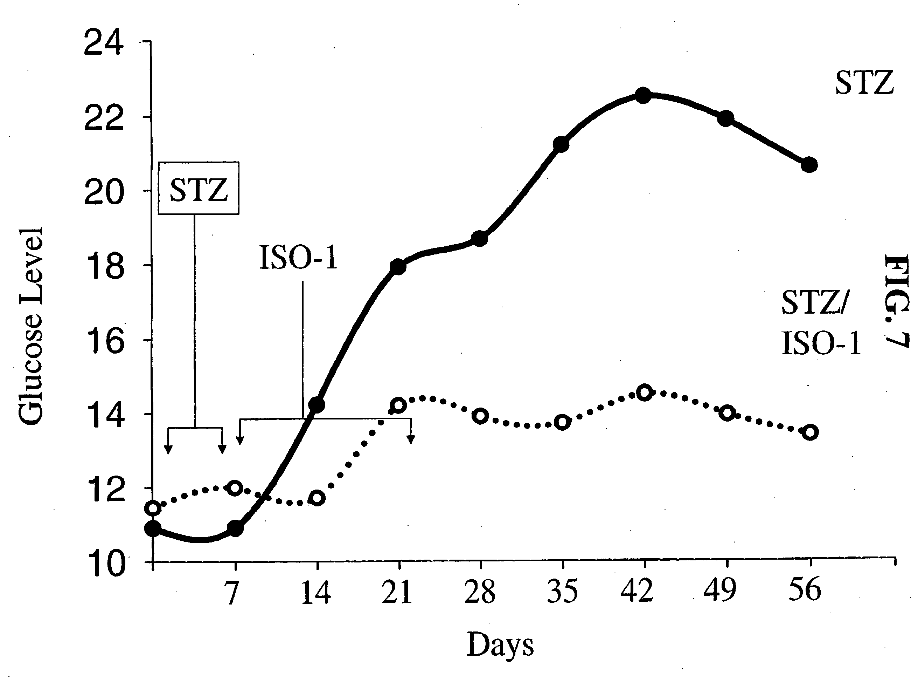

FIG. 7 is a graph of experimental results showing inhibition of diabetes in streptozotocin-treated mice that were treated with an MIF inhibitor.

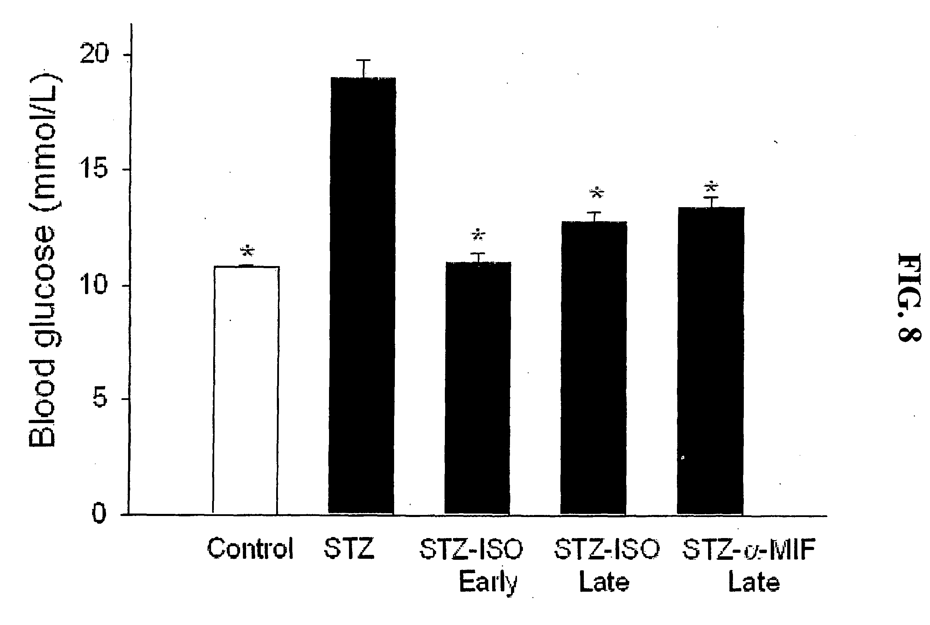

FIG. 8 is a graph of experimental results showing the effect of timing of MIF inhibitor ISO-1 and anti-MIF antibody on control of diabetes in streptozotocin-treated mice. Blood glucose levels in untreated CBA/H mice (control, n=8), or of 21 day MLD-STZ diabetic mice treated with either STZ alone (STZ, n=8), or STZ and ISO-1 given as an early (STZ-ISO Early, n=8), Late (STZ-ISO-1 Late, n=8) or Late anti-MIF Ab (STZ-•-MIF, n=8). Early injection: ISO-1 was administered by *i*.*p*. injection at 40mg/Kg/day for 14 days and started 3 days prior to STZ first injection; Late injection: ISO-1 or anti-MIF Ab was administered on day six, after the last injection of STZ. *p<0.05 refers to corresponding STZ animals.

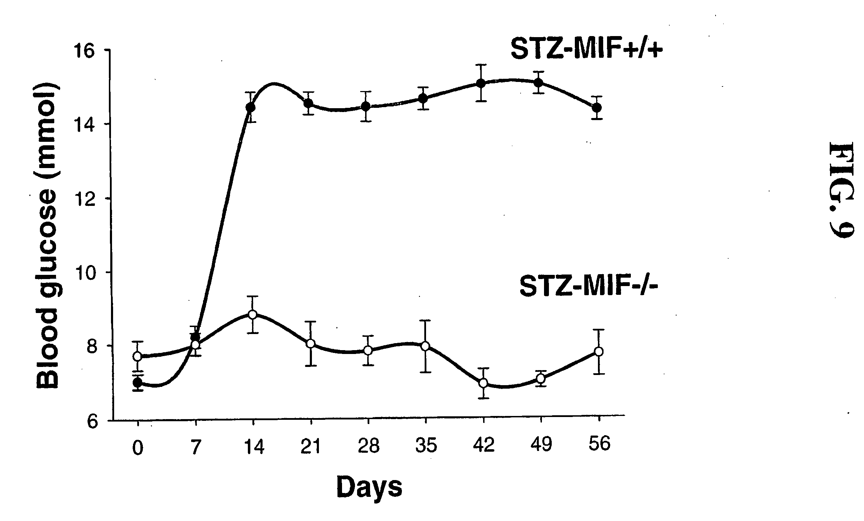

FIG. 9 is a graph of experimental results showing that MIF-null mice do not acquire diabetes after treatment with streptozotocin. Blood glucose levels were determined in wild-type and MIF^{-/-} C57B1/6 mice. Animals received MLD-STZ injections (5x40 mg/kg/day). Blood glucose levels were determined starting at the beginning of the STZ treatment and continued through weekly measurements. *p<0.005 refers to corresponding STZ animals. Blood glucose levels were determined: (a) starting at the beginning of the STZ treatment and continued through weekly measurements. Symbols used for the respective two groups which received MLD-STZ injections: C57B1/6 mice (•; n=11) and MIF-/- C57B1/6 (o; n= 11). Results from a representative of two independent experiments are presented as ± SD. *p<0.005 refers to corresponding STZ animals. Statistical analysis was performed by ANOVA with Bonferroni's adjustment.

### DETAILED DESCRIPTION OF THE INVENTION

Abbreviations: MIF, macrophage migration inhibitory factor; ISO-1, (*S*,*R*)-3-(4-hydroxyphenyl)-4,5-dihydro-5-isoxazole acetic acid methyl ester ; iNOS, inducible nitric oxide synthase; STZ, streptozotocin; MLD-STZ, multiple low doses of streptozotocin; SMNC, spleen mononuclear cells; PC, peritoneal cells; IFN-γ, γ-interferon; TNF-α, tumor necrosis factor-α; IL-1β, interleukin-1β.

The present invention is based on the discovery that MIF is a critical factor in the pathogenesis of type 1 diabetes. Therefore, inhibition of MIF prevents or attenuates the development of type 1 diabetes. See Examples.

Thus, in some embodiments, the invention is directed to an antibody that inhibits macrophage migration inhibitory factor (MIF) for use in treating a mammal having type 1 diabetes or at risk for type 1 diabetes. These uses comprise administering to the mammal a pharmaceutical composition comprising an agent that inhibits a macrophage migration inhibitory factor (MIF) in the mammal.

The antibody that binds specifically to the MIF can be an Fab fragment or an F(ab)2 fragment of an antibody. Such agents can be produced by well known methods. Non-limiting methods include: immunization of animals with MIF, followed by isolation of anti-MIF antibodies from serum or production of anti-MIF monoclonal antibodies from hybridomas made by fusion of spenocytes with myeloma cells; or phage display or other recombinant methods. Preferably, the monoclonal antibody is chosen or adapted to match the species to be treated. For treatment of humans, for example, the anti-MIF antibody (or antigen-binding fragment thereof) will be a human antibody or a humanized antibody. Such antigen-specific human or humanized monoclonal antibodies may be developed by a variety of methods well known in the art.

These methods can be used with any mammal. Preferably, the mammal is a rodent (e.g., a mouse injected with streptozotocin, which is an accepted animal model for type 1 , diabetes). In other preferred embodiments, the mammal is a human.

The invention is also directed to methods of treating a mammal having type 1 diabetes or at risk for type 1 diabetes. These methods comprise administering to the mammal a

Any of the above-described compositions can be formulated without undue experimentation for administration to a mammal, including humans, as appropriate for the particular application. Additionally, proper dosages of the compositions can be determined without undue experimentation using standard dose-response protocols.

Accordingly, the compositions designed for oral, lingual, sublingual, buccal and intrabuccal administration can be made without undue experimentation by means well known in the art, for example with an inert diluent or with an edible carrier. The compositions may be enclosed in gelatin capsules or compressed into tablets. For the purpose of oral therapeutic administration, the pharmaceutical compositions of the present invention may be incorporated with excipients and used in the form of tablets, troches, capsules, elixirs, suspensions, syrups, wafers, chewing gums and the like.

Tablets, pills, capsules, troches and the like may also contain binders, recipients, disintegrating agent, lubricants, sweetening agents, and flavoring agents. Some examples of binders include microcrystalline cellulose, gum tragacanth or gelatin. Examples of excipients include starch or lactose. Some examples of disintegrating agents include alginic acid, corn starch and the like. Examples of lubricants include magnesium stearate or potassium stearate. An example of a glidant is colloidal silicon dioxide. Some examples of sweetening agents include sucrose, saccharin and the like. Examples of flavoring agents include peppermint, methyl salicylate, orange flavoring and the like. Materials used in preparing these various compositions should be pharmaceutically pure and nontoxic in the amounts used.

The compositions useful for the present invention can easily be administered parenterally such as for example, by intravenous, intramuscular, intrathecal or subcutaneous injection. Parenteral administration can be accomplished by incorporating the compositions of the present invention into a solution or suspension. Such solutions or suspensions may also include sterile diluents such as water for injection, saline solution, fixed oils, polyethylene glycols, glycerine, propylene glycol or other synthetic solvents. Parenteral formulations may also include antibacterial agents such as for example, benzyl alcohol or methyl parabens, antioxidants such as for example, ascorbic acid or sodium bisulfite and chelating agents such as EDTA. Buffers such as acetates, citrates or phosphates and agents for the adjustment of tonicity such as sodium chloride or dextrose may also be added. The parenteral preparation can be enclosed in ampules, disposable syringes or multiple dose vials made of glass or plastic. Rectal administration includes administering the pharmaceutical compositions into the rectum or large intestine. This can be accomplished using suppositories or enemas. Suppository formulations can easily be made by methods known in the art. For example, suppository formulations can be prepared by heating glycerin to about 120° C., dissolving the composition in the glycerin, mixing the heated glycerin after which purified water may be added, and pouring the hot mixture into a suppository mold.

Transdermal administration includes percutaneous absorption of the composition through the skin. Transdermal formulations include patches (such as the well-known nicotine patch), ointments, creams, gels, salves and the like.

The present invention includes nasally administering to the mammal a therapeutically effective amount of the composition. As used herein, nasally administering or nasal administration includes administering the composition to the mucous membranes of the nasal passage or nasal cavity of the patient. As used herein, pharmaceutical compositions for nasal administration of a composition include therapeutically effective amounts of the composition prepared by well-known methods to be administered, for example, as a nasal spray, nasal drop, suspension, gel, ointment, cream or powder. Administration of the composition may also take place using a nasal tampon or nasal sponge.

In preferred embodiments of the invention, the mammal has or is at risk for having diabetes or conditions associated with diabetes or a risk of diabetes, for example impaired glucose intolerance, stress hyperglycemia, metabolic syndrome, and/or insulin resistance.

The present invention can be utilized for any mammal, for example rodents, generally used as experimental models for diabetes (see Example), or humans.

The discovery that compounds that inhibit MIF activity or expression are useful for treatment of type 1 diabetes indicates that screens of compounds for MIF-inhibiting activity can be used to identify compounds for the ability to prevent or treat type 1 diabetes. Thus, in some embodiments, the invention is directed to methods of evaluating whether a compound is useful for preventing or treating type 1 diabetes. The methods comprise the following two steps: (a) determining whether the compound inhibits a macrophage migration inhibitory factor (MIF) in a mammal, then, if the compound inhibits the MIF, (b) determining whether the compound inhibits development of type 1 diabetes.

In these methods, the ability of the tested compound to inhibit MIF activity can be determined by any known procedure. Non-limiting examples include those methods described in U.S. Patent Application Publications 2003/0008908 and 2003/0195194. Similarly, any known procedure for evaluating the effect of a compound on type 1 diabetes can be utilized. In preferred embodiments, the effect of the compound on type 1 diabetes is determined by evaluating the effect of the compound on development of diabetes in animal models utilizing multiple low dose streptozotocin administration or in animal models genetically susceptible to development of type 1 diabetes, such as the NOD mouse. The effect of a test compound or formulation on the development of diabetes in such models may be assessed by a variety of convenient methods, for instance by monitoring circulating glucose or proliferation of splenic lymphocytes in the mammal (see also Example).

These methods are not limited to evaluation of any particular type of compound. Examples of compounds that can be evaluated by these methods include oligopeptides or proteins such as enzymes or proteins that comprise an antibody binding site; nucleic acids or mimetics such as antisense compounds, ribozymes, aptamers, interfering RNAs such as siRNAs. In preferred embodiments, the compound is an organic molecule less than 1000 Dalton having structure I or structure II, as previously defined.

In additional embodiments, the invention is directed to kits comprising (a) a pharmaceutical composition comprising an agent that inhibits a macrophage migration inhibitory factor (MIF) in the mammal, where the agent is a polypeptide or a polynucleotide, and (b) instructions for administering the composition to the mammal. In these embodiments, the mammal has type 1 diabetes or is at risk for type 1 diabetes.

Preferred embodiments of the invention are described in the following examples.

### Example 1. Neutralization of Macrophage Migration Inhibitory Factor (MIF) Activity

### Attenuates Experimental Autoimmune Diabetes

### Example Summary

The pro-inflammatory cytokine, macrophage migration inhibitory factor (MIF), plays a pivotal role in several inflammatory and autoimmune diseases. MIF mRNA expression is up-regulated in non-obese diabetic mice, yet little is known about its potential as a regulator of type 1 diabetes. Here, we show that MIF protein is significantly elevated in islet cells during the development of experimental diabetes induced in mice by multiple low doses of streptozotocin. Attenuation of MIF activity with exemplary MIF inhibitors such as neutralizing antibodies against MIF, or the pharmacological MIF inhibitor (*S*,*R*)-3-(4-hydroxyphenyl)-4,5-dihydro-5-isoxazole acetic acid methyl ester (ISO-1), markedly reduces histopathological changes in the islets of pancreas and suppresses the development of hyperglycaemia, showing the general utility of this method of treating mammals with, or at risk for, type 1 diabetes. The observed beneficial effects could be attributed to the reduced proliferation and adhesion of autoreactive lymphocytes, down-regulation of iNOS expression, as well as NO and TNF-α secretion by islets of pancreas and by peritoneal macrophages, although knowledge of these mechanistic links are not essential to the teaching of the inventive method. This study defines a critical role for MIF in the pathogenesis of type 1 diabetes and identifies a new therapeutic strategy to attenuate the autoimmune process at multiple levels.

### Introduction

The observation that MIF also stimulates the synthesis of other proinflammatory cytokines and soluble mediators involved in the pathogenesis of type 1 DM such as TNF-α, IL-1β and nitric oxide (NO) (19) raised the possibility that inhibition of MIF activity may modulate the development of disease.

Given the central regulatory role of MIF in the immune response mediated by both macrophages and T cells, we have investigated the expression of MIF during the progress of diabetes in mice treated with multiple low doses of streptozotocin (MLD-STZ), as well as the influence of MIF activity neutralization on the disease development. These are well-accepted models for type 1 diabetes in humans. For this purpose, an anti-MIF polyclonal antibody, as well as (S,R)3-(4-hydroxyphenyl)-4,5-dihydro-5-isoxazole acetic acid methyl ester (ISO-1), a pharmacological inhibitor of MIF (20), were given to mice as prophylactic treatment prior to the challenge with STZ. Various immunological parameters relevant for DM pathogenesis were determined, including autoreactive cell proliferation and adhesion, as well as the production of proinflammatory mediators. To our knowledge, this is the first attempt to interfere in autoimmune diabetes by *in vivo* neutralization of MIF.

### Materials and Methods

Mice. Inbred CBA/H mice were obtained from our own breeding colony at the Institute for Biological Research, Belgrade. Inbred C57 B1/6 mice were originally purchased from Charles River (Calco, Italy) and then bred by brother per sister mating for up to 4 generations. Mice were kept under standard laboratory conditions with free access to food and water. The handling of the mice and the study protocol were approved by the local Institutional Animal Care and Use Committee.

Reagents. Streptozotocin (STZ, S-0130), sulfanilamide, naphthylenediamine dichidrochloride and irrelevant rabbit IgG were purchased from Sigma (St. Louis, MO). Anti-murine MIF IgG was prepared from rabbit serum raised against murine MIF and purified by protein A affinity chromatography following the manufacturer's instructions (Pierce, Rockford, IL). ISO-1, [(S,R)-3-(4-hydroxyphenil)-4,5-dihydro-5-isoxasole acetic acid methyl ester] was synthesized as previously described (20).

Diabetes induction and *in vivo* treatments. Diabetes was induced in adult male mice with multiple subtoxic doses of streptozotocin (MLD-STZ, 40 mg/kg body wt/day i.p. for five consecutive days) as described (21). The impact of polyclonal antibody against MIF was studied by i.p. injection of mice (5-10 per treatment group) with 5 mg/kg of rabbit IgG antibody against mouse MIF on days -3, -1, 2 and 5 in relation to the first STZ dose. The effect of ISO-1 was studied by i.p. injection of the drug at a dose of 1 mg/mouse/day, for 14 consecutive days, starting 3 days prior to the first injection with STZ. Control animals received either nonimmune IgG or ISO-1 diluent (DMSO/H₂O). Mice were monitored for diabetes by weekly measurement of blood glucose levels using a glucometer (Sensimac^{®}, Imaco, Ludersdorf, Germany). Clinical diabetes was defined by hyperglycemia in non-fasted animals (blood glucose over 11.8 mmol/l).

Cell incubation and determination of pro-inflammatory mediators. Resident peritoneal cells (PC), spleen mononuclear cells (SMNC), and pancreatic islets were isolated from individual anti-MIF IgG-treated, ISO-1-treated or control diabetic mice on day 15 after the first injection of STZ, as well as from normal untreated animals. SMNC (5x10⁶/well) prepared by Ficoll gradient centrifugation, resident PC (2.5x10⁵/well) collected by peritoneal lavage with cold PBS, or pancreatic islets (150-200 islets/well) prepared by collagenase digestion and density gradient purification as described (22), were incubated in 24-well Limbro culture plates in 1 ml of RPMI-1640 culture medium containing 5% fetal bovine serum (FBS) and cell supernatants were collected after 48 h. Concentration of bioactive TNF-α in culture supernatant was determined as previously described (22) using a cytolytic bioassay with actinomycin D-treated fibrosarcoma cell line L929. Nitrite accumulation, an indicator of NO production, was determined in cell culture supernatant using the Griess reaction (22). The expression of intracellular MIF, IFN-γ, MHC II, or iNOS was determined by slight modification of cell-based ELISA protocol (23). Spleen MNC (5x10⁵/well) or PC (2.5x10⁵/well), were allowed to adhere to poly-L-lysine-precoated 96-well microplates. Following fixation with 4% paraformaldehyde, and washing with 0.1 % Triton X-100 in PBS (T/PBS), endogenous peroxidase was quenched with 1% H₂O₂ in T/PBS, and reaction blocked for 1 h at 37°C with 10% FBS in T/PBS. Subsequently, cells were incubated for 1 h at 37°C with either rabbit anti-mouse MIF IgG, rat anti-mouse IFN-γ (Holland Biotechnology, Leiden, The Netherlands), mouse anti-mouse/rat MHC II (MCA46R, Serotec), or rabbit anti-mouse iNOS (Sigma), in T/PBS containing 1% BSA. After washing, the cells were incubated for 1 h with the corresponding secondary antibody (goat anti-rabbit Ig(H+L)-HRP, goat anti-rat Ig(H+L)-HRP or goat anti-mouse Ig(F(ab')₂-specific)-HRP washed again and incubated for 15 min at room temperature in dark with 50 µl of a solution containing 0.4 mg/ml OPD (Sigma), 11.8 mg/ml Na₂HPO₄x2H₂=O, 7.3 mg/ml citric acid and 0.015% H₂O₂. The reaction was stopped with 3N HCl, and the absorbance was measured in a microplate reader at 492 nm in a Titertek microplate reader (Flow).

*Ex vivo* lymphoproliferative response and adhesion assay. Spontaneous proliferation of SMNC was determined by incubation of cells (5x10⁵/well) from each individual animal in 96-well microplates with 1 pCi ³H-thymidine (³H-TdR, ICN). Incorporated radioactivity was measured after 24 h in a Beckman liquid scintillation counter. The analysis of spontaneous adhesion of SMNC (2.5x10⁵/well) to a monolayer of L929 fibroblasts or plastic was performed by using crystal violet assay as previously described (21). The absorbance corresponding to the number of adherent cells, was measured at 570 nm.

Antibodies and flow cytometry. Spleen MNC (1x10⁶) were incubated with the rat anti-mouse monoclonal antibodies (mAbs): anti-CD11b (MAC-1)-phycoerythrin (PE), or anti-CD25 (IL-2 Receptor α chain, p55)-biotin (PharMingen, San Diego, CA), followed by Streptavidin-PE (PharMingen). Each cell suspension of SMNC was a pool from three to five animals obtained from the same experimental group 15 days after diabetes induction, as well as from normal untreated mice. Cell surface marker expression was analyzed with a flow cytometer (FACScalibur, Becton Dickinson, Heidelberg, Germany) and Win MDI 2.8 software (Joseph Trotter).

Histological and immunohistochemical examination of pancreas. Pancreata were fixed in neutral buffered formalin and then embedded in paraffin. The fixed blocks were sectioned (7 µm thick) and haematoxylin and eosin staining was performed to assess the incidence and degree of inflammatory changes. Insulitis scoring was performed as previously described (10, 11) by examining at least 15 islets per mouse and graded in a blinded fashion as follows: 0, no infiltrate; 1, peri-ductal infiltrate; 2, peri-islet infiltrate; 3, intraislet infiltrate; and 4, intraislet infiltrate associated with β-cell destruction. At least 15 islets were counted for each mouse. A mean score for each pancreas was calculated by dividing the total score by the number of islets examined. Insulitis scores (IS) are expressed as mean values ± SD. Immunohistochemistry was performed on paraffin-embedded sections of formalin-fixed tissue using a previously described microwave-based method (24). For MIF immunostaining, a polyclonal rabbit anti-MIF IgG and control rabbit IgG were used. The examined area of the islet was outlined, and the percentage of MIF-positive islet cells was measured using quantitative Image Analysis System (Optima 6.5, Media Cybernatics, Silver Springs, MD).

Statistical analysis. The blood glucose values are shown as mean values ± SE. Statistical analyses were performed by ANOVA with Bonferroni's adjustment and Fisher's exact test. The other values were expressed as means ± SD and groups of data were compared using Student's paired t-test. The results of a representative of at least three separate experiments with similar results are presented. Statistical significance was set at P<0.05.

### Results

Increased MIF expression in MLD-STZ-induced diabetic mice. Recent studies have revealed that MIF mRNA expression is up-regulated in spontaneously diabetic NOD mice, but its functional role in disease progression is unknown. In order to determine if MIF protein expression levels are altered during immunoinflammatory diabetes, MLD-STZ was administered to diabetes-susceptible CBA/H mice. Immunohistochemistry revealed substantial induction of MIF protein expression by the islet cells in pancreas sections from these mice during the disease course, as well as mononuclear cell infiltration (FIG. 1B-D). Likewise, higher concentrations of MIF were detected in the peritoneal cells (PC) of diabetic mice, in comparison to non-diabetic, control mice (FIG. 1E). *In vivo* treatment of mice with neutralizing anti-MIF immunoglobulin during disease induction significantly suppressed PC expression of MIF (FIG. 1E). Thus, as a consequence of DM induction, increased MIF-protein content was observed both at the level of peripheral and target tissue, and MIF protein levels can be attenuated by administration of anti-MIF antibodies.

Anti-MIF prophylaxis suppresses clinical and histological parameters of MLD-STZ-induced diabetes. To determine whether inhibition of MIF activity modulates disease in MLD-STZ-exposed mice, we first studied the effect of a neutralizing polyclonal antibody against MIF in two DM-susceptible inbred mouse strains. Both C57B1/6 and CBA/H control mice treated with PBS or non-immune IgG developed sustained hyperglycemia over a 2-week period following MLD-STZ injections. Although MLD-STZ induced different degrees of hyperglycemia in the two mouse strains, treatment of either C57B1/6 mice (FIG. 2A), or CBA/H mice (FIG. 2B) with anti-MIF Ab from day -3 to day +5 significantly inhibited MLD-STZ-induced hyperglycemia. In addition, histological examination of pancreatic specimens performed on day 15 of the disease indicated that the degree of islet infiltration was significantly lower in mice treated with anti-MIF antibodies as compared with diabetic control mice treated with irrelevant IgG (FIG. 2C and D). We also examined the effect of the pharmacological MIF antagonist ISO-1 (20) in MLD-STZ-induced DM. Accordingly, we treated mice prophylactically over a period of 14 days with ISO-1, starting 3 days before the first of five injections of STZ. These mice remained euglycemic throughout the eight-week experimental period (FIG. 2B). The anti-diabetogenic effect of either immunological or pharmacological neutralization of MIF was long-lasting, with limited variations throughout the entire 56 day follow-up period (FIG. 2A and B). MIF blockade by ISO-1 attenuated inflammation of the islets (FIG. 2D). These data provide evidence for a critical role of MIF in the induction and progression of immunoinflammatory DM and for the utility of treatment with MIF inhibitors to avert the development of type 1 diabetes in at-risk populations.

Anti-MIF treatments reduce splenocyte proliferation and adhesive properties. To understand the cellular effects of anti-MIF treatments, *ex vivo* analysis of the functional characteristics of spleen mononuclear cells (SMNC) from CBA/H mice was performed during early progression of hyperglycemia, on day 15 of hyperglycemia. Previous studies have shown that isolated autoreactive lymphocytes from diabetic mice exhibit significantly increased *ex vivo* spontaneous proliferation in comparison to cells isolated from healthy animals, suggesting that this cell type may contribute to disease progression (21, 22). To determine if MIF inhibitors modulate splenocyte proliferative responses *ex vivo,* diabetic CBA/H mice were treated with either anti-MIF antibodies or ISO-1, euthanized at day 15 after MLD-STZ exposure, and splenocytes harvested for *ex vivo* analyses. Treatment of MLD-STZ-exposed mice with anti-MIF antibodies significantly inhibited diabetes-associated SMNC proliferation (³H-thymidine incorporation was 29782 ± 3694 cpm *versus* 63651 ± 10157 cpm of diabetic control cells) (FIG. 3A). Likewise, *in vivo* inhibition of MIF activity with ISO-1 reverted splenocyte proliferation to near-control levels (25637 ± 2015 cpm in comparison to 21150 ± 12723 cpm of untreated control cells) (FIG. 3A). In addition to cell proliferation, cell-cell adhesion, mediated by the interaction between CD11b and ICAM-1 (CD54), participates in the immunological processes of type 1 DM (21, 25). To determine possible consequences of anti-MIF treatment on the interactions between cells participating in the diabetogenic processes, we assessed the adhesive properties of SMNC and found that spontaneous adhesion of cells to plastic, as well as adhesion to fibroblasts was significantly inhibited by *in vivo* treatment with anti-MIF IgG, or ISO-1 (FIG. 3B). Down-regulation of adhesiveness was associated with the changes of CD11b expression, as revealed by flow cytometry analysis, using antibodies directed against CD11b, a molecule that mediates cellular adhesion to ICAM-1 (CD54). Thus, in comparison to SMNC derived from control diabetic mice, both anti-MIF IgG treatment and ISO-1 treatment (Table 1) reduced the frequency of CD11b⁺ SMNC and mean antigen density (mean fluorescence intensity, MFI) to the level of untreated normal mice, indicative of the anti-diabetogenic benefits of treatment with the MIF inhibitors.

**Table 1. MIF antagonists down-regulate the expression of CD11b and CD25 of splenic mononuclear cells**

| Treatment groups | | CD11b⁺ | | CD25⁺ | |
|---|---|---|---|---|---|
| | | %^{*} | MFI** | % | MFI |
| Exp. 1 | | | | | |
| | Untreated | 7.9 | 44.4 | 4.4 | 57.8 |
| | STZ + IgG | 9.4 | 72.0 | 8.5 | 77.7 |
| | STZ + αMIF | 6.4 | 48.6 | 4.3 | 70.6 |
| | | | | | |

| Exp. 2 | | | | | |
|---|---|---|---|---|---|
| | Untreated | 15.5 | 88.1 | 2.5 | 96.2 |
| | STZ | 19.4 | 108.3 | 6.1 | 113.0 |
| | STZ + ISO-1 | 15.3 | 88.5 | 4.5 | 106.0 |

Data of two out of five experiments with similar results obtained by flow cytometry analysis from the pull of three mouse spleens per group. 'Frequency of positive cells. **Mean fluorescence intensity.

Since IL-2 is centrally involved in the initiation of any immune response, negating its action by blocking the interaction with the IL-2 receptor (IL-2R, CD25) system has gained much attention as a possible therapeutic target for immunointervention in both rodent and human disease (26). Anti-CD25 has also been shown to attenuate low-dose streptozotocin-induced diabetes in mice (12, 26). To determine the effect of *in vivo* treatment with MIF antagonists on IL-2R⁺ cell populations, lymphocytes were isolated from the spleens of control or anti-MIF-treated mice 15 days post-MLD-STZ exposure and labeled with fluorescently tagged antibodies directed against IL-2R (CD25). Staining the cells for IL-2R expression (Table 1) revealed significant increase in both the percentage of IL-2R⁺ lymphocytes and antigen density in control diabetic mice, when comparing to normal untreated animals, while the *in vivo* treatment with anti-MIF IgG, or with ISO-1 reduced these parameters.

Production of pro-inflammatory mediators. Since pro-inflammatory mediators play crucial role in type 1 DM development in rodents (4-7, 21, 22), we determined the effects of MIF inhibition on STZ-associated cytokine release from both local and peripheral immune cells *ex vivo*. Immunological neutralization of endogenous MIF protein with anti-MIF IgG (FIG. 4A), or pharmacological inhibition of MIF with ISO-1 (FIG. 4B), down-regulated TNF-α production by spleen MNC, peritoneal macrophages and pancreatic islet cells, reducing it to the level of healthy, nontreated mice. We also found that intracellular expression of iNOS was significantly reduced in macrophages isolated from animals treated with either anti-MIF or ISO-1 in comparison to relatively high iNOS expression of diabetic animals (FIG. 5A). Accordingly, MIF blockade abolished subsequent NO production in macrophages, as well as in pancreatic islets (FIG. 5B). The effects of the MIF inhibitors were specific for TNF-α, iNOS, and NO, because the expression of intracellular IFN-γ in the splenocytes, as well as the expression of MHC class II molecules, remained unchanged in comparison to control diabetic mice (FIG. 6A and B).

### Discussion

MIF significantly contributes to the immunopathology associated with excessive inflammation and autoimmunity, and neutralizing endogenous MIF with neutralizing anti-MIF antibodies, or targeted disruption of the MIF gene, inhibits the development of several rodent models of inflammatory disease, including immunologically induced kidney disease (27), leishmaniasis (28), Gram-negative and Gram-positive sepsis (15, 29, 30), antigen-induced arthrius (31), colitis (16), and experimental autoimmune encephalomyelitis (EAE) (17). Recently, a potential role for MIF in the development and pathogenesis of autoimmune-mediated DM has been implicated in spontaneously diabetic NOD mice, because expression of MIF mRNA is significantly increased during disease development, and exogenous MIF administration increases disease incidence in these animals (18). MIF is constitutively expressed and secreted together with insulin from pancreatic β-cells, and acts as an autocrine factor to stimulate insulin release (32). Because induction of insulin secretion is thought to contribute to immunoinflammatory diabetogenic pathways by favoring the expression on the β-cells and the presentation to the immune cells of antigens that are up-regulated when the functional activity is augmented (12), this "hormonal" property could represent an additional important factor involving endogenous MIF in the initial events of β-cell dysfunction and destruction. Targeting endogenous MIF may therefore be a suitable approach for unraveling the role of this cytokine in the pathogenesis of type 1 DM and for therapeutic and/or prophylactic treatment of the condition.

In the present study, we show for the first time that endogenous MIF plays a key role in the development of murine autoimmune diabetes. Progression of MLD-STZ-induced diabetes was accompanied by up-regulated MIF protein expression both in pancreatic islets and peripheral cells. Immunoneutralization of MIF by anti-MIF IgG, or pharmacological inhibition of MIF activity with ISO-1, markedly attenuated the clinical and histological manifestations of the disease. It inhibited the inflammatory responses as well as splenocyte proliferation and adherence *ex vivo*. The anti-diabetogenic effect of both agents was long-lasting, because mice remained euglycemic during the 8-week follow-up period. Inhibition of autoreactive T cell proliferation, leukocyte adhesion and migration into target tissue, and macrophage activation by these anti-MIF strategies suggest that immunological or pharmacological neutralization of MIF activity may attenuate pathologic autoimmune responses *in vivo*.

Consistent with the role of MIF in the T-cell activation and mitogenesis (30, 33), we found that MIF blockade considerably decreased IL-2R expression and spontaneous proliferation of splenic lymphocytes measured ex vivo, which most probably reflected the autoreactive T cell response to blood-born pancreatic antigens released upon STZ destruction of β-cells (21, 22). The results therefore indicate that MIF contributes to the clonal size of autoreactive T cells, and that blocking the expansion of diabetogenic T clones might be partly responsible for the protective effect of these anti-MIF strategies.

In addition to clonal expansion of autoreactive cells, the development of the disease is determined by cell-cell interactions mediated by adhesion receptors and ligands both in the induction and the efferent phase of the autoimmune response. In this context, we have shown that mononuclear cells from MLD-STZ-induced diabetic mice are highly adherent to cultured vascular endothelial cells (21), or fibroblasts (FIG. 3B) at a time of peak disease activity; these adhesive properties may be an important mechanism for inflammatory infiltration of target tissue (21). Several lines of evidence demonstrate that up-regulation of CD11b, which mediates cellular adhesion to ICAM-1 (CD54) (34), might have functional consequences on the interactions between cells participating in the immunological processes of type 1 DM (21, 25). MIF blockade significantly inhibited the expression of CD11b on mononuclear cells of diabetic mice and markedly down-regulated leukocyte adhesive properties, suggesting that anti-MIF strategies may impair homing of these cells to the islets of pancreas, thus contributing to the therapeutic benefit of MIF blockade. In favor of this hypothesis, histological analysis showed reduced insulitis in anti-MIF treated mice. Inhibition of MIF activity also has been shown to down-regulate adhesion molecule-dependent target tissue pathology in EAE (17).

Another important finding of this study is that anti-MIF treatment affects the production of pro-inflammatory and cytotoxic mediators. The lower local production of TNF-α and NO may result from the reduction of inflammatory cell influx into pancreas. Based on our *ex vivo* findings, however, it seems that MIF blockade might also directly influence macrophage and T cell effector function, as suggested by down-regulation of TNF-α, iNOS and NO by peritoneal and/or spleen cells. This is consistent with the *in vitro* ability of anti-MIF antibodies to interfere with production of these mediators (28). MIF up-regulates in vitro production of TNF-α, as well as of reactive oxygen species and nitrogen metabolites (35). It therefore is conceivable that direct blockade of MIF-mediated iNOS expression and TNF-α synthesis may contribute to down-regulation of tissue-damaging pro-inflammatory mediators in type 1 DM. Thus, we hypothesize that the efficacy of MIF blockade in the treatment of MLD-STZ DM is due to inhibitory effects on the autoimmune/inflammatory response of T cells and macrophage, as well as islet cells.

Interestingly, the effect of systemic blockade of MIF specifically inhibited TNF-α and iNOS-mediated NO production in both local and peripheral cells, because these therapeutic approaches did not diminish peripheral production of the signature Th1 cytokine, IFN-γ. Consistent with the role for IFN-γ in the regulation of MHC class II, the expression of antigens by peritoneal and spleen cells was not impaired after anti-MIF treatments. Although these observations appear to contradict previous findings demonstrating a central role for IFN-γ in diabetes pathogenesis (36), it must be noted that peripheral cytokine production may not mirror the role of cytokine in the development of the organ-specific autoimmune disease. For example we have previously shown in the DP-BB rat model that in an apparently paradoxical fashion, both anti-IFN-γ antibody and exogenously-administered IFN-γ prevented development of autoimmune diabetes (10, 37). As recently reported for EAE, anti-MIF antibody treatment did not affect antigen-specific splenocyte production of IFN-γ, but did reduce IFN-γ-release from antigen-reactive Th1 cells in the target tissue (17). Therefore, it is possible that anti-MIF treatment in our model reduced cytokine production within the microenvironment of the pancreas, but this effect may not have been reflected in our *ex vivo* analyses. In support of this, we have previously shown a similar modulatory pattern of IFN-γ expression and distribution of MHC class II+ cells in MLD-STZ-induced type 1 DM by different systemically applied pharmacological agents (21, 22). It is known that IFN-γ may possess dicothomic effects on inflammation, exerting proinflammatory effects when produced at the level of the organ targeted by the immune response (e.g. the islet microenvironment), while activating corticosteroid-dependent and independent anti-inflammatory pathways at the systemic level (8). Having in mind the profound ability of MIF to counteract the immunosuppressive effects of glucocorticoids (38), neutralization of MIF activity accompanied with sustained circulating levels of IFN-γ could potentiate systemic anti-inflammatory effects in the treated animals.

MIF is a critical mediator of inflammatory and autoimmune diseases, because neutralizing endogenous MIF activity with anti-MIF antibodies has been effective in animal models of septic shock, colitis, encephalomyelitis, and leishmania infection (15-17, 28), and may be a promising approach for therapy of various human diseases. However, treatment approaches that rely on exogenously administered proteins, including humanized antibodies, may face several challenges, including potential immunogenicity, that suggest the desirability of more preferred embodiments. Anti-cytokine antibodies can form small inflammatory complexes with cytokines, and thereby exacerbate inflammatory responses (39). For these reasons, alternatives to the therapeutic use of anti-MIF Ig preparations should also be considered for pharmaceutical development. Thus, pharmacological intervention with ISO-1, as a small drug-like molecule that inhibits MIF tautomerase and biological activity (20), may be a more preferred approach for the treatment of MIF-related diseases. Future studies will address the potential use of MIF inhibitors as a treatment, rather than a prophylactic, for regulation of diabetes pathogenesis and presentation.

### Example 2. Delayed MIF inhibition after induction of diabetes (comparative)

Using methods as described in Example 1, diabetes was induced in adult male mice with multiple subtoxic doses of streptozotocin (MLD-STZ, 40 mg/kg body wt/day i.p. for five consecutive days) as described (21). The effect of MIF inhibition after induction of diabetes was studied by i.p. injection of (S,R)-3-(4-hydroxyphenyl)-4,5-dihydro-5-isoxazole acetic acid methyl ester (ISO-1), a pharmacological inhibitor of MIF (20) at a dose of 1 mg/mouse/day, for 14 consecutive days starting at day 7 after initial streptozotocin administration. Peripheral blood glucose was monitored weekly in these mice and in control mice that were treated identically, except where the ISO-1 treatment was replaced by ISO-1 diluent.

As shown in FIG. 7 and 8, inhibition of MIF after induction of diabetes provided considerable benefit to the streptozotocin-treated mice, by inhibiting the progression of diabetes.

### Example 3 MIF-null Mice are Resistant to the Induction of Type 1 Diabetes by Streptozotocin

To further establish the importance of MIF in type 1 diabetes, MIF-/- C57B1/6 mice (40) were treated with streptozotocin and compared with MIF^{+/+} mice in progression of diabetes. When treated with streptozotocin, the MIF-/- mice did not develop diabetes, while the MIF+/+ mice did develop diabetes (FIG. 9). This also further establishes that methods directed to inhibiting expression of MIF would be expected to be useful for type 1 diabetes treatment.

### SEQ ID Nos

SEQ ID NO:1 - Human MIF cDNA - GenBank Accession No. NM002415 
1 accacagtgg tgtccgagaa gtcaggcacg tagctcagcg gcggccgcgg cgcgtgcgtc
61 tgtgcctctg cgcgggtctc ctggtccttc tgccatcatg ccgatgttca tcgtaaacac
121 caacgtgccc cgcgcctccg tgccggacgg gttcctctcc gagctcaccc agcagctggc
181 gcaggccacc ggcaagcccc cccagtacat cgcggtgcac gtggtcccgg accagctcat
241 ggccttcggc ggctccagcg agccgtgcgc gctctgcagc ctgcacagca tcggcaagat
301 cggcggcgcg cagaaccgct cctacagcaa gctgctgtgc ggcctgctgg ccgagcgcct
361 gcgcatcagc ccggacaggg tctacatcaa ctattacgac atgaacgcgg ccaatgtggg
421 ctggaacaac tccaccttcg cctaagagcc gcagggaccc acgctgtctg cgctggctcc
481 acccgggaac ccgccgcacg ctgtgttcta ggcccgccca ccccaacctt ctggtgggga
541 gaaataaacg gtttagagac t
SEQ ID NO:2 - Mouse MIF cDNA - GenBank Accession No. BC024895 
1 ggcttgggtc acaccgcgct ttgtaccgtc ctccggtcca cgctcgcagt ctctccgcca
61 ccatgcctat gttcatcgtg aacaccaatg ttccccgcgc ctccgtgcca gaggggtttc
121 tgtcggagct cacccagcag ctggcgcagg ccaccggcaa gcccgcacag tacatcgcag
181 tgcacgtggt cccggaccag ctcatgactt ttagcggcac gaacgatccc tgcgccctct
241 gcagcctgca cagcatcggc aagatcggtg gtgcccagaa ccgcaactac agtaagctgc
301 tgtgtggcct gctgtccgat cgcctgcaca tcagcccgga ccgggtctac atcaactatt
361 acgacatgaa cgctgccaac gtgggctgga acggttccac cttcgcttga gtcctggccc
421 cacttacctg caccgctgtt ctttgagcct cgctccacgt agtgttctgt gtttatccac
481 cggtagcgat gcccaccttc cagccgggag aaataaatgg tttataagag aaaaaaaaaa
541 aaaaaaa
SEQ ID NO:3 - Rat MIF cDNA - GenBank Accession No. NM031051

1 gggtcacgta gtcaggtccc agacttgggt cacaccgcac ttaacaccgt cctccggccg
61 tcgttcgcag tctctccgcc accatgccta tgttcatcgt gaacaccaat gttccccgcg
121 cctccgtgcc agaggggttt ctctccgagc tcacccagca gctggcgcag gccaccggca
181 agccggcaca gtacatcgca gtgcacgtgg tcccggacca gctcatgact tttagtggca
241 cgagcgaccc ctgcgccctc tgcagcctgc acagcatcgg caagatcggt ggcgcccaga
301 accgcaacta cagcaagctg ctgtgcggcc tgctgtccga tcgcctgcac atcagcccgg
361 accgggtcta catcaactat tacgacatga acgcagccaa cgtgggctgg aacggttcca
421 ccttcgcttg agcccgggcc tcacttacct gcaccgctgt tcttcgagtc ttgctgcacg
481 ccccgttctg tgtttatcca cccgtaatga tggccacctt ccggtcggga gaaataaatg
541 gtttgagacc a

## Claims

1. An antibody that inhibits macrophage migration inhibitory factor (MIF) for use in inhibiting the progression of type 1 diabetes in a mammal having type 1 diabetes or for inhibiting the development of type 1 diabetes in a mammal at risk for type 1 diabetes.

2. The antibody for use of claim 1, for inhibiting the progression of type 1 diabetes in a mammal having type 1 diabetes.

3. The antibody for use of claim 1, for inhibiting the development of type 1 diabetes in a mammal at risk for type 1 diabetes.

4. The antibody for use of claim 1, wherein the antibody is a monoclonal antibody.

5. The antibody for use of claim 1, wherein the antibody is a humanized antibody.

6. The antibody for use of claim 1, wherein the antibody is a human antibody.

7. The antibody for use of any of the preceding claims,
wherein the antibody is an Fab fragment or an F(ab)₂ fragment.

8. The antibody for use of any of the preceding claims,
wherein the mammal has or is at risk for impaired glucose tolerance, stress hyperglycemia, metabolic syndrome, and/or insulin resistance.

9. The antibody for use of any of the preceding claims, wherein the mammal is a rodent.

10. The antibody for use of any of the preceding claims, wherein the mammal is a human.

## Patentansprüche

1. Ein Antikörper zur Hemmung des Macrophage Inhibitory Factor (MIF) zur Verwendung in der Hemmung des Fortschreitens von Typ I Diabetes in einem Säuger mit Typ 1 Diabetes oder zur Hemmung der Entwicklung von Typ 1 Diabetes in einem Säuger mit einem Risiko für Typ 1 Diabetes.

2. Der Antikörper zur Verwendung nach Anspruch 1, zur Hemmung des Fortschreitens von Typ I Diabetes in einem Säuger mit Typ 1 Diabetes.

3. Der Antikörper zur Verwendung nach Anspruch 1, zur Hemmung der Entwicklung von Typ 1 Diabetes in einem Säuger mit einem Risiko für Typ1 Diabetes.

4. Der Antikörper zur Verwendung nach Anspruch 1, worin der Antikörper ein monoklonaler Antikörper ist.

5. Der Antikörper zur Verwendung nach Anspruch 1, worin der Antikörper ein humanisierter Antikörper ist.

6. Der Antikörper zur Verwendung nach Anspruch 1, worin der Antikörper ein humaner Antikörper ist.

7. Der Antikörper zur Verwendung nach einem der vorhergehenden Ansprüche, worin der Antikörper ein Fab-Fragment oder ein F(ab)₂-Fragment ist.

8. Der Antikörper zur Verwendung nach einem der vorhergehenden Ansprüche, worin der Säuger verminderte Glukosetoleranz, stressbedingte Hyperglykämie, das metabolische Syndrom, und/oder Insulinresistenz hat oder ein Risiko für diese besitzt.

9. Der Antikörper zur Verwendung nach einem der vorhergehenden Ansprüche, worin der Säuger ein Nager ist.

10. Der Antikörper zur Verwendung nach einem der vorhergehenden Ansprüche, worin der Säuger ein Mensch ist.

## Revendications

1. Anticorps qui inhibe le facteur d'inhibition de la migration des macrophages (MIF) à utiliser pour inhiber la progression du diabète de type 1 chez un mammifère ayant le diabète de type 1 ou pour inhiber le développement du diabète de type 1 chez un mammifère risquant de présenter le diabète de type 1.

2. Anticorps à utiliser selon la revendication 1, pour inhiber la progression du diabète de type 1 chez un mammifère ayant le diabète de type 1.

3. Anticorps à utiliser selon la revendication 1, pour inhiber le développement du diabète de type 1 chez un mammifère risquant de présenter le diabète de type 1.

4. Anticorps à utiliser selon la revendication 1, où l'anticorps est un anticorps monoclonal.

5. Anticorps à utiliser selon la revendication 1, où l'anticorps est un anticorps humanisé.

6. Anticorps à utiliser selon la revendication 1, où l'anticorps est un anticorps humain.

7. Anticorps à utiliser selon l'une quelconque des revendications précédentes, où l'anticorps est un fragment Fab ou un fragment F(ab)₂.

8. Anticorps à utiliser selon l'une quelconque des revendications précédentes, où le mammifère a ou risque de développer une tolérance altérée au glucose, une hyperglycémie de stress, un syndrome métabolique et/ou une résistance à l'insuline.

9. Anticorps à utiliser selon l'une quelconque des revendications précédentes, où le mammifère est un rongeur.

10. Anticorps à utiliser selon l'une quelconque des revendications précédentes, où le mammifère est un humain.
